# EUROPEAN PATENT SPECIFICATION

(11) **EP 0 257 133 B1**
(45) Date of publication and mention of the grant of the patent: **27.01.1993**
(21) Application number: 86114792.4
(22) Date of filing: 24.10.1986
(51) Int. Cl.: A61F 13/00

(54) **Wound dressing and process for making same**
Wundverband und Verfahren zu seiner Herstellung
Pansement pour blessures et son procédé de fabrication

(30) Priority: 25.08.1986 US 899575
(43) Date of publication of application: 02.03.1988
(73) Proprietor: The B.F. GOODRICH Company, Akron, Ohio 44318 (US)
(72) Inventor: Ewall, Ralph Xavier, Chagrin Falls Ohio 44022 (US)
(74) Representative: von Kreisler, Alek, Dipl.-Chem.

(56) References cited:
- BE-A- 821 734
- DE-A- 2 837 894
- FR-A- 2 226 979
- US-A- 3 255 748
- US-A- 4 145 464

## Description

The present invention relates to wound dressings, and pertains more particularly to wound dressings primarily for application over wounds which would be expected to exude substantial amounts of fluid during the normal healing process.

Exudation from many types of skin lesions is normal during the healing process. Included are such wounds as Stage II and Stage III ulcers, second and third degree burns, skin grafts and donor sites, deep dermabrasions and lacerations. Conventional bandages or wound dressings readily absorb fluids, when used for such lesions, and soon become saturated with exudate seeping from the open wound, necessitating frequent bandage or dressing changes. The frequent changes cause irritation of the wound, discomfort to the patient and increased health care costs. A wound dressing which would have the capability of absorbing exudate as it appears at the surface of an open wound and the ability to transmit absorbed moisture at a desired rate through the dressing to the distal surface of the dressing (the surface furthest removed from the wound when the dressing is in place over the wound) where it can evaporate from the dressing would be particularly useful as a wound covering. Since such dressing could remain in place over the wound without needing to be changed for a longer period of time than conventional dressings, the wound would not be disturbed as frequently as a result of dressing changes and the patient would not experience as much discomfort from the associated trauma.

Various attempts have been made to provide a wound dressing that is capable of more effectively absorbing exudate seeping from an open wound.

In US-A-3,339,546, a dressing comprised of a water-impervious film having an adhesive layer plied with the water-impervious film is described. The adhesive layer is disclosed as being a blend of a water-soluble or water-swellable hydrocolloid material dispersed throughout a water-insoluble viscous elastic binder.
US-A-3,255,743 discloses a wound dressing (surgical moleskin) comprising a fabric layer (woven cotton) having inward and distal broadside faces, a flexible layer hydrophilic material (intermediate woven fabric) disposed between said broadside faces of said fabric layer, and a layer of non-toxic pressure-sensitive adhesive (natural or synthetic rubber adhesive, positioned on one side of said fabric layer for adhering said dressing onto the patient, the layers of said wound dressing being sterilizable .

US-A-3,972,328 describes a wound dressing consisting of three components, namely, (1) a middle layer comprised of a semi-open cell flexible foam, (2) a water-impervious flexible film attached over one surface of the middle layer, and (3) a pressure-sensitive adhesive layer disposed over the other surface of the middle layer. The pressure-sensitive adhesive component is formed of a pressure-sensitive elastomer containing a water-soluble or water-swellable hydrocolloid or mixture of such hydrocolloids and contains, additionally, a tackifier and a plasticizer or solvent.

Although the use of water-soluble or water-swellable hydrocolloids dispersed in the adhesive layer of a wound dressing allows wound exudate to be absorbed by the hydrocolloids, the absorption of the exudate by the particles of hydrophilic material, with attendant swelling of the particles, prematurely destroys the integrity of the adhesive film resulting in the film's disintegration.

### SUMMARY OF THE INVENTION

The present invention provides a wound dressing that is able to absorb significant quantities of wound exudate emitted from an open wound without the integrity of the dressing being destroyed and, in the preferred construction, has the capability of allowing moisture contained in the exudate to be transferred within a desired rate through the dressing to the distal surface of the dressing where it can evaporate into the surrounding atmosphere, thereby lengthening the useful life of the dressing. In accordance with this invention, a wound dressing is provided that includes a layer of fabric in which a flexible essentially continuous film of a hydrophilic material is deposited. A layer of a pressure-sensitive adhesive material is uniquely adhered to one side of the fabric for affixing the dressing to the patient, the adhesive material being one that will adhere to the healthy skin of the patient, but tends not to adhere to the open wound itself. To provide additional integrity to the dressing, an exterior cover layer is provided that forms the distal surface of the dressing, i.e., the surface of the dressing furthest removed from the wound when the dressing is in place over the wound. The various component layers of the dressing preferably have a porosity such that moisture contained in the body fluid can pass through the dressing at a desired rate and be evaporated at the distal face of the dressing.

### DESCRIPTION OF THE DRAWINGS

Fig. 1 is a perspective view of a segment of a wound dressing embodying the present invention;
Fig. 2 is a perspective view of a segment of a second embodiment of this invention;
Fig. 3 is a schematic view illustrating forming a composite component of the wound dressings of Figs. 1 and 2;
Fig. 4 is a schematic view illustrating combining together two composite components to form the wound dressing shown in Fig. 1; and
Fig. 5 is a schematic view illustrating combining together two composite components with an adhesive layer to form the wound dressing of Fig. 2.

### DETAILED DESCRIPTION OF THE INVENTION

In detail, the present invention pertains to a wound dressing to manage wound fluids by rapidly absorbing wound exudate and thereby minimizing skin maceration, which wound dressing comprises a laminate containing the following layers:
(a) an adhesive layer (12, 22) capable of permitting passage of wound fluid therethrough, said adhesive layer upon pressure contact with the skin of a patient permitting prolonged adhesion of the wound dressing to said skin without adhering to the wound;
(b) a fabric layer (11, 21) bonded to said adhesive layer (12, 22) which fabric layer retains structural integrity upon exposure of said wound dressing to wound exudate; and
(c) a cover layer (17, 27) forming the distal surface of said wound dressing:
said wound dressing being characterized by having a hydrophilic absorbent polymeric layer (15, 24) comprising hydrophilic absorbent material deposited essentially entirely within said fabric layer (11, 21), said hydrophilic absorbent polymer layer being capable of absorbing the liquid drawn into said wound dressing from said wound exudate when said wound dressing is placed over the wound of a patient.

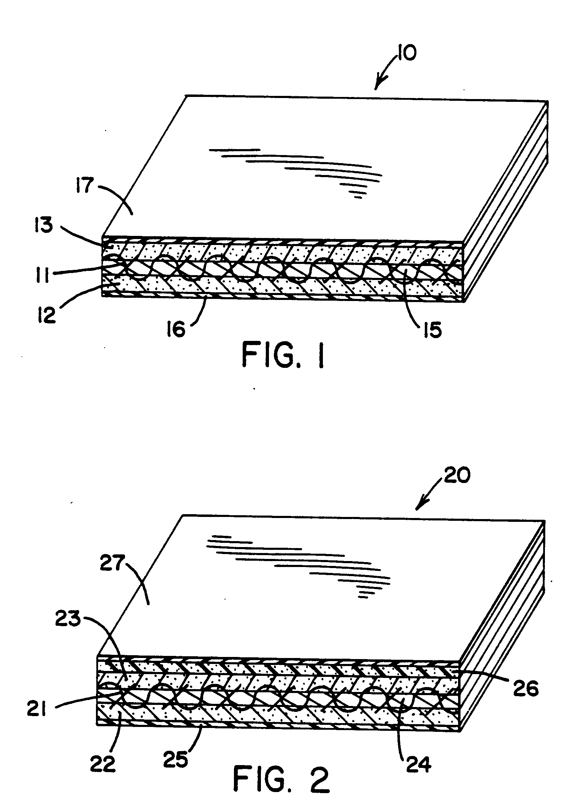

The wound dressing 10, shown in Fig. 1, is comprised of a fabric layer 11 plied with and adhered to a layer 12 of an adhesive material over one of its broadside faces (its inward broadside face) and a second layer 13 of an adhesive material over its other broadside face (its distal broadside face). Adhesive layers 12 and 13 desirably are pressed into fibers protruding outwardly from the surfaces of the fabric layer 11 so that the adhesive becomes mechanically bonded with the fiber ends protruding from the body of fabric layer 11. A layer 15 of a flexible hydrophilic material is disposed between adhesive layers 12 and 13 and within fabric layer 11. A release layer 16 is disposed over and releasably secured to the exposed broadface surface of adhesive layer 12 to protect adhesive layer 12 during storage of dressing 10, layer 16 being removed just prior to the application of dressing 10 over the wound. A protective cover layer 17 is disposed exteriorly over fabric layer 11 and is bonded to fabric layer 11 by adhesive layer 13. Cover layer 17 not only serves as an outer protective layer providing a high degree of structural integrity to the dressing 10, but it also can function as a shield that prevents contaminants from gaining access to the interior of the dressing and, ultimately, to the wound.

Fabric layer 11 desirably is a flexible high-lofted, non-toxic fabric that has sufficient structural integrity to withstand normal handling, processing and use. Fabric layer 11 can be formed of any non-toxic fibers, such as cotton, nylon, rayon, polyester, and polyester cellulose fibers, and, if a non-woven fabric, it desirably is of a spun-bonded or spun-laced construction, although wet-laid or air-laid structures can be employed. The fabric desirably has numerous fibers protruding from its broadside faces to facilitate a secure mechanical bond between fabric layer 11 and adhesive layers 12 and 13. An example of a fabric that has been found to perform exceptionally well in wound dressings of the present invention are 101.6 to 355.6 µm (4 to 14 mil) thick spun-bonded polyester staple fiber fabrics sold by E.I. duPont de Nemours & Company under the trade designation "Reemay."

Adhesive layer 12 may be formed of any pressure-sensitive, non-toxic, adhesive suitable for adhesion to healthy normal human skin. Although adhesive layer 12 will adhere to normal healthy skin, the adhesive has little, if any, tendency to adhere to the open wound itself and, therefore, minimizes any potential interference with normal healing, and minimizes trauma to the neoepithelium on removal of the dressing. In addition to being non-toxic, adhesive layer 12 desirably should be sterilizable by any conventional means (such as radiation, thermal or steam processes, although sterilization by gamma ray or electron beam irradiation is preferred), non-cytotoxic, non-sensitizing and have a moisture vapor transmission rate (MVTR) of at least 200 grams of water, preferably between 300 to 800 grams of water, per square meter per 24 hours at 50% relative humidity at 36°C when measured in accordance with ASTM Procedure No. E96-80. Suitable pressure-sensitive skin adhesive compositions are pressure-sensitive polyacrylic skin adhesives (such as are available from Daubert Coated Products Company, and Fasson Division of Avery International Corporation, and Semex Medical of Seton Company), pressure-sensitive polyvinyl ether adhesives, and pressure-sensitive polyurethane adhesives.

Adhesive layer 13 may be formed of the same adhesive composition as adhesive layer 12 or it may be formed of a different adhesive composition, provided that it is sterilizable by conventional processes and desirably has an MVTR of at least 200 grams of water, but preferably between 300 to 800 grams of water, per square meter per 24 hours at 50% relative humidity at 36°C when measured in accordance with ASTM Procedure No. E96-80.

The flexible layer 15 of hydrophilic material disposed within fabric layer 11 and between adhesive layers 12 and 13 may be any flexible non-toxic hydrophilic material that is capable of retaining its integrity even after absorbing 2 to 20 times its weight of exudate. Such hydrophilic materials include sodium carboxymethylcellulose, various polyacrylamide, polyacrylonitrile and acrylic acid polymers, Karaya gum and polysaccarides. A non-toxic flexible film layer 15 formed of a cross-linked acrylic acid polymer prepared by photo- or radiation-polymerizing (in a nitrogen atmosphere) 90 to 99.9 weight percent of acrylic acid, 50 to 100% of the carboxylic groups having been neutralized with an alkali metal hydroxide or ammonium hydroxide prior to polymerization, with 0.1 to 10 percent of a polyfunctional cross-linking agent and 0.1 to 5 weight percent of a photo-initiator based on the total weight of acrylic acid and cross-linking agent, such polymers being more fully described in Canadian Patent No. 1,160,984 issued January 24, 1984, has been found to be particularly effective. Other useful hydrophilic materials are those described in U.S. Patent Nos. 4,062,817 and 4,066,583.

Cover layer 17 may be formed of any non-toxic material that is sterilizable by conventional processes and desirably has an MVTR of at least 200 grams of water, preferably between 300 to 800 grams of water, per square meter per 24 hours at 50% relative humidity at 36°C when measured in accordance with ASTM Procedure No. E96-80. Desirably, cover layer 17 is from 12.7 µm to 50.8 µm (0.5 to 2.0 mils) thick and typically may be polyester polyurethane, a polyether polyurethane, polyethylene, copolyester, or polyether block imide film.

To provide dressing 10 with the ability to transmit moisture through the dressing 10 at an MVTR of between 300 to 800 grams of water per square meter per 24 hours at 50% relative humidity at 36°C when measured in accordance with ASTM Procedure No. E96-80, it is necessary that all component layers of dressing 10 have an MVTR of at least 300 grams of water per square meter per 24 hours at 50% relative humidity at 36°C and that at least one of the layers, and preferably cover layer 17, have an MVTR of between 300 to 800 grams of water per square meter per 24 hours at 50% relative humidity at 36°C when measured in accordance with ASTM Procedure No. E96-80.

Wound dressing 20, shown in Fig. 2, is comprised of a fabric layer 21 with a layer 22 of a pressure-sensitive adhesive material over one of its broadside faces and a second layer 23 of an adhesive material over its other broadside face. Like adhesive layers 12 and 13 of dressing 10, adhesive layers 22 and 23 of dressing 20 desirably are pressed into fibers protruding outwardly from the respective broadside faces of fabric layer 21 with which they are associated to promote mechanical bonds between adhesive layers 22 and 23 and fabric layer 21. A layer 24 of a flexible hydrophilic material is disposed between adhesive layers 22 and 23 and within fabric layer 21. A releasable protective layer 25 is disposed over the exposed broadside face of adhesive layer 22 to protect adhesive layer 22 during storage, layer 25 being removed immediately before dressing 20 is placed over the wound. A layer 26 of an open-cell sponge material is adhered to the exterior broadside face of adhesive layer 23. Sponge layer 26 provides a degree of cushioning protection to a wound against forces externally applied against dressing 20 when dressing 20 is in place over a wound. Cover layer 27 is disposed over and adhered to layer 26 and not only can serve to impart structural integrity to dressing 20, but also can serve as a shield that prevents contaminants from gaining access to the interior of dressing 20. Layers 21, 22, 23, 24, 25 and 27 of dressing 20 correspond functionally to layers 11, 12, 13, 15, 16 and 17, respectively, of dressing 10, and may be formed of the same materials as described in regard to their respective counterparts of dressing 10.

Sponge layer 26 may be made of any flexible, open-cell cellular material that has sufficient integrity to withstand clinical handling, is sterilizable by conventional processes, and desirably has an MVTR of at least 200 grams of water per square meter per 24 hours at 50% relative humidity at 36°C when measured in accordance with ASTM Procedure No. E96-80. Layer 26 may be from about 1 to 25 mm thick, but preferably is from about 1 to 3 mm thick. Latex foam rubber, polyvinyl chloride foam, polyethylene foam and polyurethane foam materials which have from 40 to 100 pores per linear 2.54 cm (inch) (PPI) are typical cellular materials which can be used for fashioning sponge layer 26. If sponge layer 26 has an exterior skin layer, such skin layer can serve in place of cover layer 27. An 80 PPI open-cell polyurethane foam sold by General Foam Plastics Corporation or Scotfoam Co. of General Felt Industry Corporation is particularly useful for forming sponge layer 26. Layer 26 can be bonded or adhered to cover layer 27 by any suitable convenient manner such as by flame bonding, thermal bonding or through use of an adhesive layer (not shown) that can be the same adhesive material as used in adhesive layers 22 and 23 or any other compatible bonding system.

If dressing 20 is to have the capability of transmitting moisture at a rate of from 300 to 800 grams of water per square meter per 24 hours at 50% relative humidity at 36°C when measured in accordance with ASTM Procedure No. E96-80, all of the component layers of dressing 20 must have an MVTR of at least 300 grams of water per square meter per 24 hours at 50% relative humidity at 36°C and at least one of the layers, preferably cover layer 27, must have an MVTR of between 300 to 800 grams of water per square meter per 24 hours at 50% relative humidity at 36°C when measured in accordance with ASTM Procedure No. E96-80.

In the manufacture of dressing 10, a continuous sheet of a composite "A" may be formed by combining continuous sheets of fabric layer 11, adhesive layer 12 and release layer 16 together in any convenient manner. For example, a continuous sheet of adhesive layer 12 supported on release layer 16 can be applied onto one broadside face of a continuous sheet of fabric layer 11. The three components then can be passed between pressure rolls (not shown) to force fibers protruding from the broadside face of fabric layer 11 opposing adhesive layer 12 into adhesive layer 12 to form a firm mechanical bond between fabric layer 11 and adhesive layer 12. The continuous sheet of composite "A" then can be wound on a roll 30 for storage and later use.

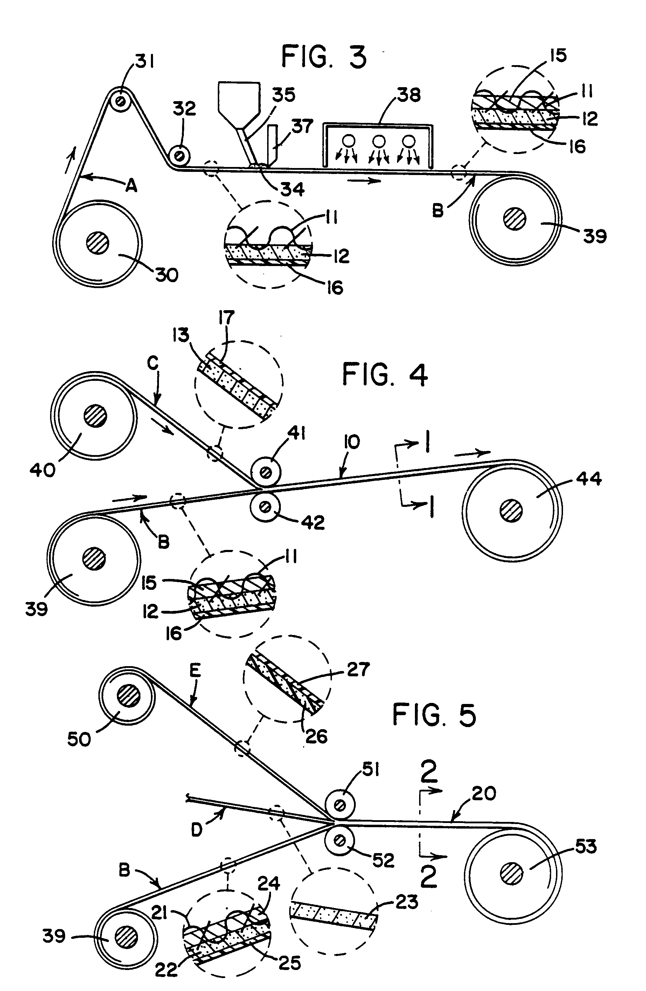

As schematically shown in Fig. 3, the layer of hydrophilic material can be formed within fabric layer 11 by unwinding the sheet of composite A from roll 30, passing the sheet of composite A over tensioning rolls 31 and 32 and advancing the sheet of composite A with the fabric layer 11 facing up beneath a spray or bank 34 of hydrophilic film-producing material emitted by nozzle 35. Composite A with the hydrophilic film-producing material deposited thereon then passes beneath doctor blade 37. Doctor blade 37 forces the hydrophilic film-producing material into the central region of fabric layer 11 and controls the thickness of the resultant hydrophlilic film (15). The composite then is passed through a drying oven or a polymerization chamber 38 depending upon whether the hydrophilic film-producing material used merely needs to be dried or whether it needs to be polymerized. If the hydrophilic film-producing material is one that requires polymerization such as those described in Canadian Patent No. 1,160,984, polymerization chamber 38 may contain sources of electron beams or ultraviolet (UV) light energy to cause the hydrophilic film-producing material to polymerize. The resulting continuous sheet of composite "B" consisting of fabric layer 11, hydrophilic film layer 15 deposited within fabric layer 11, adhesive layer 12 and release layer 16 then can be wound upon a roll 39 for storage.

A continuous sheet of a composite "C" consisting of adhesive layer 13 and cover layer 17 may be formed by combining continuous sheets of adhesive layer 13 and cover layer 17 in any convenient manner. For example, a continuous sheet of adhesive layer 13 can be applied over cover layer 17. Then, the two layers can be passed between pressure rolls (not shown) to form the composite. The continuous sheet of composite C then can be wound on a roll 40 for storage and later use.

The sheets of composite B and composite C then can be combined with adhesive layer 13 of composite C in facing relationship with fabric layer 11 of composite B, as schematically shown in Fig. 4. The combined sheets of composite B and composite C then are passed between pressure rolls 41, 42 to cause the protruding fibers of fabric layer 11 to be pressed into adhesive layer 13 to form a mechanical bond between fabric layer 11 and adhesive layer 13 thereby completing the manufacture of wound dressing 10 except for cutting and packaging the composite sheet in dressings of desired size. In the interim, the continuous sheet of dressing 10 can be wound on roll 44. Of course, if desired, the process of making wound dressing 10 can be a continuous type of operation.

Wound dressing 20 may be manufactured, as schematically illustrated in Fig. 5. A continuous sheet of composite B (as described above) wound on roll 39 is plied with a continuous sheet of adhesive "D" which is a sheet of the adhesive material that forms adhesive layer 23 of dressing 20, and a continuous sheet of composite "E" wound on roll 50. Composite E consists of sponge layer 26 bonded to cover layer 27 either by flame bonding, thermal bonding or by a layer of adhesive material (not shown). In combining composites B and E with adhesive layer D, the exposed face of fabric layer 21 is brought in contact with one face of adhesive sheet D while the exposed face of sponge layer 26 is brought in contact with the opposite face of adhesive sheet D. The assembly then is passed between the nip of pressure rolls 51, 52 which causes adhesive sheet D to bond both to sponge layer 26 and to fabric layer 21 to form the dressing 20. The completed dressing 20 can be wound onto roll 53 to await being cut into convenient sizes and packaged. Again, the various steps of assembling dressing 20 can be performed as a continuous operation.

Desirably, the wound dressings have oxygen permeability when wetted by wound exudate to provide transmission of at least 2000, preferably 5000 cm³ of oxygen per square meter per 24 hours at 25°C and relative humidity between 25 to 55%, when measured in accordance with ASTM Procedure No. D3985-81.

## Claims (Claims for the following Contracting State(s): AT, LI, FR, IT, SE, BE, DE, GB, LU, CH, GR, NL)

1. A wound dressing to manage wound fluids by rapidly absorbing wound exudate and thereby minimizing skin maceration, which wound dressing comprises a laminate containing the following layers:
(a) an adhesive layer (12, 22) capable of permitting passage of wound fluid therethrough, said adhesive layer upon pressure contact with the skin of a patient permitting prolonged adhesion of the wound dressing to said skin without adhering to the wound;
(b) a fabric layer (11, 21) bonded to said adhesive layer (12, 22) which fabric layer retains structural integrity upon exposure of said wound dressing to wound exudate; and
(c) a cover layer (17, 27) forming the distal surface of said wound dressing,
said wound dressing being characterized by having a hydrophilic absorbent polymeric layer (15, 24) comprising hydrophilic absorbent material deposited essentially entirely within said fabric layer (11, 21), said hydrophilic absorbent polymer layer being capable of absorbing the liquid drawn into said wound dressing from said wound exudate when said wound dressing is placed over the wound of a patient."

2. The wound dressing of claim 1, wherein said fabric layer (11, 21) has protruding fibers embedded into and mechanically bonded with said adhesive layer (12, 22).

3. The wound dressing of claims 1 and 2, wherein, said hydrophilic absorbent material is a cross-linked acrylic and is cured from a monomer solution disposed within said fabric layer (11, 21).

4. The wound dressing of claims 1 and 2, wherein said fabric layer (11, 21) is composed of a polyester which is spun-bonded, spun-laced or point-bonded.

5. The wound dressing of claims 1 and 2, wherein the components of said wound dressing all have a moisture vapor transmission rate (MVTR) when measured in accordance with ASTM Procedure No. E96-80 of at least 200 grams of water, preferably at least 300 grams of water, per square meter per 24 hours at 50% relative humidity at 36°C.

6. The wound dressing of claims 1 and 2, wherein said wound dressing has a release layer (16, 25) releasably adhered to the exposed surface of said adhesive layer (12, 22).

7. The wound dressing of claims 1 and 2, wherein said wound dressing has a layer of flexible open-cell cellular material disposed between said fabric layer (11, 21) and cover layer (17, 27) and adhesively bonded thereto.

8. The process of making a wound dressing comprised of a fabric layer, a pressure-sensitive adhesive layer disposed over and adhered to a broadside face of said fabric layer, a release layer disposed over and releasably adhered to said adhesive layer, a second adhesive layer disposed over and adhered to the other broadside face of said fabric layer, a flexible layer of hydrophilic material disposed within said fabric layer between said first and second layers of adhesive material and a cover layer disposed as the distal face of said wound dressing, said process comprising:
a. combining in adherent relationship a sheet of said fabric layer material and a sheet of said release layer material with a sheet of the first said adhesive layer material disposed therebetween,
b. introducing a layer of hydrophilic-forming material into said layer of fabric and converting said layer of hydrophilic forming material into a hydrophilic layer disposed within said fabric layer,
c. disposing said second layer of adhesive material in adherent relationship over the exposed broadside face of the fabric layer, and
d. assembling said cover layer as the distal component of said wound dressing.

9. The process of claim 8 wherein a layer of open-cell cellular material is adherently disposed between said cover layer and said second-mentioned layer of adhesive material.

10. The process of claim 8 wherein said hydrophilic-forming material is polymerized using radiant energy to produce a layer of hydrophilic material.

11. The process of claim 8 wherein said cover layer is disposed in adherent relationship over the second-mentioned layer of adhesive material.

## Claims (Claims for the following Contracting State(s): ES)

1. Process for making a wound dressing to manage wound fluids by rapidly absorbing wound exudate and thereby minimizing skin maceration, which wound dressing comprises a laminate containing the following layers:
(a) an adhesive layer (12, 22) capable of permitting passage of wound fluid therethrough, said adhesive layer upon pressure contact with the skin of a patient permitting prolonged adhesion of the wound dressing to said skin without adhering to the wound;
(b) a fabric layer (11, 21) bonded to said adhesive layer (12, 22) which fabric layer retains structural integrity upon exposure of said wound dressing to wound exudate; and
(c) a cover layer (17, 27) forming the distal surface of said wound dressing,
said wound dressing being characterized by having a hydrophilic absorbent polymeric layer (15, 24) comprising hydrophilic absorbent material deposited essentially entirely within said fabric layer (11, 21), said hydrophilic absorbent polymer layer being capable of absorbing the liquid drawn into said wound dressing from said wound exudate when said wound dressing is placed over the wound of a patient said process comprising:
a. combining in adherent relationship a sheet of said fabric layer material and a sheet of said release layer material with a sheet of the first said adhesive layer material disposed therebetween,
b. introducing a layer of hydrophilic-forming material into said layer of fabric and converting said layer of hydrophilic forming material into a hydrophilic layer disposed within said fabric layer,
c. disposing said second layer of adhesive material in adherent relationship over the exposed broadside face of the fabric layer, and
d. assembling said cover layer as the distal component of said wound dressing.

2. The process of claim 1 wherein said fabric layer (11, 21) has protruding fibers embedded into and mechanically bonded with said adhesive layer (12, 22)

3. The process of claims 1 and 2, wherein, said hydrophilic absorbent material is a cross-linked acrylic and is cured from a monomer solution disposed within said fabric layer (11, 21).

4. The process of claims 1 and 2, wherein said fabric layer (11, 21) is composed of a polyester which is spun-bonded, spun-laced or point-bonded.

5. The process of claims 1 and 2, wherein the components of said wound dressing all have a moisture vapor transmission rate (MVTR) when measured in accordance with ASTM Procedure No. E96-80 of at least 200 grams of water, preferably at least 300 grams of water, per square meter per 24 hours at 50% relative humidity at 36°C.

6. The process of claims 1 and 2, wherein said wound dressing has a release layer (16, 25) releasably adhered to the exposed surface of said adhesive layer (12, 22).

7. The process of claims 1 and 2, wherein said wound dressing has a layer of flexible open-cell cellular material disposed between said fabric layer (11, 21) and cover layer (17, 27) and adhesively bonded thereto.

8. The process of making a wound dressing comprised of a fabric layer, a pressure-sensitive adhesive layer disposed over and adhered to a broadside face of said fabric layer, a release layer disposed over and releasably adhered to said adhesive layer, a second adhesive layer disposed over and adhered to the other broadside face of said fabric layer, a flexible layer of hydrophilic material disposed within said fabric layer between said first and second layers of adhesive material and a cover layer disposed as the distal face of said wound dressing, said process comprising:
a. combining in adherent relationship a sheet of said fabric layer material and a sheet of said release layer material with a sheet of the first said adhesive layer material disposed therebetween,
b. introducing a layer of hydrophilic-forming material into said layer of fabric and converting said layer of hydrophilic forming material into a hydrophilic layer disposed within said fabric layer,
c. disposing said second layer of adhesive material in adherent relationship over the exposed broadside face of the fabric layer, and
d. assembling said cover layer as the distal component of said wound dressing.

9. The process of claim 8 wherein a layer of open-cell cellular material is adherently disposed between said cover layer and said second-mentioned layer of adhesive material.

10. The process of claim 8 wherein said hydrophilic-forming material is polymerized using radiant energy to produce a layer of hydrophilic material.

11. The process of claim 8 wherein said cover layer is disposed in adherent relationship over the second-mentioned layer of adhesive material.

## Patentansprüche (Patentansprüche für folgende(n) Vertragsstaat(en): AT, BE, CH, DE, FR, GB, GR, IT, LI, LU, NL, SE)

1. Wundverband zur Behandlung von Wundflüssigkeiten durch schnelles Absorbieren von Wundexsudaten und dadurch Minimieren von Hautmazeration, wobei der Wundverband eine Schichtung umfaßt, die folgende Lagen enthält:
(a) eine Klebeschicht (12, 22), die befähigt ist, Wundflüssigkeit durch dieselbe hindurchtreten zu lassen, wobei die Klebeschicht auf Druckberührung mit der Haut des Patienten ein verlängertes Kleben des Wundverbands an der Haut erlaubt, ohne an der Wunde zu kleben;
(b) eine Gewebeschicht (11, 21), die mit der Klebeschicht (12, 22) verklebt ist, wobei die Gewebeschicht ihre Strukturbeständigkeit behält, wenn der Wundverband dem Wundexsudat ausgesetzt wird; und
(c) eine Deckschicht (17, 27), die die abgewandte Oberfläche des Wundverbands bildet,
wobei der Wundverband dadurch gekennzeichnet ist, daß er eine hydrophile absorbierende Polymerschicht (15, 24) hat, umfassend hydrophiles absorbierendes Material, das im wesentlichen vollständig in die Gewebeschicht (11, 21) eingelagert ist, wobei die hydrophile absorbierende Polymerschicht befähigt ist, die aus dem Wundexsudat in den Wundverband gezogene Flüssigkeit zu absorbieren, wenn der Wundverband auf die Wunde eines Patienten gelegt ist.

2. Wundverband nach Anspruch 1, worin die Gewebeschicht (11, 21) heraushängende Fasern hat, die in die Klebeschicht eingebettet sind und mechanisch mit der Klebeschicht (12, 22) verklebt sind.

3. Wundverband nach Anspruch 1 und 2, worin das hydrophile absorbierende Material aus vernetztem Acryl besteht und aus einer monomeren Lösung, die in die Gewebeschicht (11, 21) eingelassen ist, ausgehärtet ist.

4. Wundverband nach Anspruch 1 und 2, worin die Gewebeschicht (11, 21) aus Polyester zusammengesetzt ist, der vliesverfestigt, spunlaced oder punktverklebt ist.

5. Wundverband nach Anspruch 1 und 2, worin alle Bestandteile des Wundverbands eine Feuchtigkeits-Dampf-Transmissions-Rate (MVTR), wenn sie nach dem ASTM Verfahren Nr. E96-80 gemessen werden, von wenigstens 200 g Wasser, vorzugsweise wenigstens 300 g Wasser pro Quadratmeter, pro 24 Stunden bei einer relativen Luftfeuchtigkeit von 50% bei 36 °C haben.

6. Wundverband nach Anspruch 1 und 2, worin der Wundverband eine Trennschicht (16, 25) hat, die trennbar an die freie Oberfläche der Klebeschicht (12, 22) geklebt ist.

7. Wundverband nach Anspruch 1 und 2, worin der Wundverband eine Schicht aus flexiblem, offenzelligem Schaumstoff hat, der zwischen die Gewebeschicht (11, 21) und die Deckschicht (17, 27) gelegt und an dieselbe geklebt ist.

8. Verfahren zur Herstellung eines Wundverbands, umfassend eine Gewebeschicht, eine druckempfindliche Klebeschicht, die über die Gewebeschicht gelegt und an eine Breitseitenoberfläche der Gewebeschicht geklebt ist, eine Trennschicht, die über die Klebeschicht gelegt und trennbar an die Klebeschicht angeklebt ist, eine zweite Klebeschicht, die über die Gewebeschicht gelegt und an die andere Breitseitenoberfläche der Gewebeschicht angeklebt ist, eine flexible Schicht aus hydrophilem Material, das in die Gewebeschicht zwischen die erste und zweite Schicht des klebenden Materials eingelagert ist, und eine Deckschicht, die als Gegenfläche des Wundverbands aufgelegt ist, wobei das Verfahren umfaßt:
a. das Verbinden einer Lage des Gewebeschichtmaterials und einer Lage des Trennschichtmaterials mit einer Lage des erst genannten Klebeschichtmaterials, das sich zwischen diesen befindet, zu einer eng haftenden Verbindung,
b. das Einführen einer Schicht eines hydrophilen Materials in die Gewebeschicht und das Umwandeln des Hydrophilie bildenden Materials in eine hydrophile Schicht, die in die Gewebeschicht eingelagert ist,
c. das Einlagern der zweiten Klebematerialschicht zu einer eng haftenden Verbindung über die freie Breitseitenoberfläche der Gewebeschicht, und
d. das Anbringen der Deckschicht als der von dem Wundverband abgewandten Komponente.

9. Verfahren nach Anspruch 8, worin eine Schicht aus offenzelligem Schaumstoff, klebend zwischen Deckschicht und der als zweite erwähnten Schicht des Klebematerials angebracht ist.

10. Verfahren nach Anspruch 8, worin das Hydrophilie bildende Material unter Verwendung von Strahlenenergie polymerisiert wird, um eine Schicht aus hydrophilem Material zu bilden.

11. Verfahren nach Anspruch 8, worin die Deckschicht in eng haftender Verbindung über die zweite erwähnte Schicht des Klebematerials angebracht ist.

## Patentansprüche (Patentansprüche für folgende(n) Vertragsstaat(en): ES)

1. Verfahren zur Herstellung eines
Wundverbands zur Behandlung von Wundflüssigkeiten durch schnelles Absorbieren von Wundexsudaten und dadurch Minimieren von Hautmazeration, wobei der Wundverband eine Schichtung umfaßt, die folgende Lagen enthält:
(a) eine Klebeschicht (12, 22), die befähigt ist, Wundflüssigkeit durch dieselbe hindurchtreten zu lassen, wobei die Klebeschicht auf Druckberührung mit der Haut des Patienten ein verlängertes Kleben des Wundverbands an der Haut erlaubt, ohne an der Wunde zu kleben;
(b) eine Gewebeschicht (11, 21), die mit der Klebeschicht (12, 22) verklebt ist, wobei die Gewebeschicht ihre Strukturbeständigkeit behält, wenn der Wundverband dem Wundexsudat ausgesetzt wird; und
(c) eine Deckschicht (17, 27), die die abgewandte Oberfläche des Wundverbands bildet,
wobei der Wundverband dadurch gekennzeichnet ist, daß er eine hydrophile absorbierende Polymerschicht (15, 24) hat, umfassend hydrophiles absorbierendes Material, das im wesentlichen vollständig in die Gewebeschicht (11, 21) eingelagert ist, wobei die hydrophile absorbierende Polymerschicht befähigt ist, die aus dem Wundexsudat in den Wundverband gezogene Flüssigkeit zu absorbieren, wenn der Wundverband auf die Wunde eines Patienten gelegt ist, wobei das Verfahren umfaßt:
a. das Verbinden einer Lage des Gewebeschichtmaterials und einer Lage das Trennschichtmaterials mit einer Lage des erst genannten Klebeschichtmaterials, das sich zwischen diesen befindet, zu einer eng haftenden Verbindung,
b. das Einführen einer Schicht eines hydrophilen Materials in die Gewebeschicht und das Umwandeln des Hydrophilie bildenden Materials in eine hydrophile Schicht, die in die Gewebeschicht eingelagert ist,
c. das Einlagern der zweiten Klebematerialschicht zu einer eng haftenden Verbindung über die freie Breitseitenoberfläche der Gewebeschicht, und
d. das Anbringen der Deckschicht als der von dem Wundverband abgewandten Komponente.

2. Verfahren nach Anspruch 1, worin die Gewebeschicht (11, 21) heraushängende Fasern hat, die in die Klebeschicht eingebettet sind und mechanisch mit der Klebeschicht (12, 22) verklebt sind.

3. Wundverband nach Anspruch 1 und 2, worin das hydrophile absorbierende Material aus vernetztem Acryl besteht und aus einer monomeren Lösung, die in die Gewebeschicht (11, 21) eingelassen ist, ausgehärtet ist.

4. Wundverband nach Anspruch 1 und 2, worin die Gewebeschicht (11, 21) aus Polyester zusammengesetzt ist, der vliesverfestigt, spunlaced oder punktverklebt ist.

5. Wundverband nach Anspruch 1 und 2, worin alle Bestandteile des Wundverbands eine Feuchtigkeits-Dampf-Transmissions-Pate (MVTR), wenn sie nach dem ASTM Verfahren Nr. E96-80 gemessen werden, von wenigstens 200 g Wasser, vorzugsweise wenigstens 300 g Wasser pro Quadratmeter, pro 24 Stunden bei einer relativen Luftfeuchtigkeit von 50% bei 36 °C haben.

6. Wundverband nach Anspruch 1 und 2, worin der Wundverband eine Trennschicht (16, 25) hat, die trennbar an die freie Oberfläche der Klebeschicht (12, 22) geklebt ist.

7. Wundverband nach Anspruch 1 und 2, worin der Wundverband eine Schicht aus flexiblem, offenzelligem Schaumstoff, der zwischen die Gewebeschicht (11, 21) und die Deckschicht (17, 27) gelegt und an dieselbe geklebt ist.

8. Verfahren zur Herstellung eines Wundverbands, umfassend eine Gewebeschicht, eine druckempfindliche Klebeschicht, die über die Gewebeschicht gelegt und an eine Breitseitenoberfläche der Gewebeschicht geklebt ist, eine Trennschicht, die über die Klebeschicht gelegt und trennbar an die Klebeschicht angeklebt ist, eine zweite Klebeschicht, die über die Gewebeschicht gelegt und an die andere Breitseitenoberfläche der Gewebeschicht angeklebt ist, eine flexible Schicht aus hydrophilem Material, das das in die Gewebeschicht zwischen die erste und zweite Schicht des klebenden Materials eingelagert ist, und eine Deckschicht, die als Gegenfläche des Wundverbands gelegt ist, wobei das Verfahren umfaßt:
a. das Verbinden einer Lage des Gewebeschichtmaterials und einer Lage des Trennschichtmaterials mit einer Lage des erst genannten Klebeschichtmaterials, das sich zwischen diesen befindet, zu einer eng haftenden Verbindung,
b. das Einführen einer Schicht eines hydrophilen Materials in die Gewebeschicht und das Umwandeln des Hydrophilie bildenden Materials in eine hydrophile Schicht, die in die Gewebeschicht eingelagert ist,
c. das Einlagern der zweiten Klebematerialschicht zu einer eng haftenden Verbindung über die freie Breitseitenoberfläche der Gewebeschicht, und
d. das Anbringen der Deckschicht als der von dem Wundverband abgewandten Komponente.

9. Verfahren nach Anspruch 8, worin eine Schicht aus offenzelligem Schaumstoff, klebend zwischen Deckschicht und der als zweite erwähnten Schicht des Klebematerials angebracht ist.

10. Verfahren nach Anspruch 8, worin das Hydrophilie bildende Material unter Verwendung von Strahlenenergie polymerisiert wird, um eine Schicht aus hydrophilem Material zu bilden.

11. Verfahren nach Anspruch 8, worin die Deckschicht in eng haftender Verbindung über die zweite erwähnte Schicht des Klebematerials angebracht ist.

## Revendications (Revendications pour l'(les) Etat(s) contractant(s) suivant(s): AT, FR, IT, SE, BE, DE, GB, LU, CH, LI, GR, NL)

1. Un pansement pour blessures pour maîtriser les fluides de la blessure par une absorption rapide des exsudats de la blessure et de manière à minimiser ainsi la macération de la peau, ledit pansement pour blessures comprenant un stratifié contenant les couches suivantes :
(a) une couche adhésive (12, 22) susceptible de laisser passer le fluide de la plaie au travers, ladite couche adhésive, lors de son contact par pression sur la peau d'un patient, assurant une adhérence prolongée du pansement pour blessure sur ladite peau, sans adhérer à la plaie ;
(b) une couche de tissu (11, 21) collée sur ladite couche adhésive (12, 22), ladite couche de tissu conservant son intégrité structurelle lors de l'exposition dudit pansement à l'exsudat de la plaie ; et
(c) une couche de recouvrement (17, 27) formant la surface distale dudit pansement pour blessures, ledit pansement pour blessures étant caractérisé en ce qu'il comporte une couche de polymère absorbant hydrophile (15, 24) conprenant un matériau absorbant hydrophile déposé essentiellement en totalité dans ladite couche de tissu (11, 21), ladite couche de polymère absorbant hydrophile étant susceptible d'absorber le liquide attiré dans ledit pansement pour blessures, provenant dudit exsudat de la blessure, lorsque ledit pansement est appliqué sur la blessure d'un patient.

2. Le pansement pour blessures selon la revendication 1, dans lequel ladite couche de tissu (11, 21) comporte des fibres en saillie noyées à l'intérieur et liées mécaniquement à ladite couche adhésive (12, 22).

3. Le pansement pour blessures selon les revendications 1 et 2, dans lequel ledit matériau absorbant hydrophile est une résine acrylique réticulée et est durci par une solution de monomère disposée dans ladite couche de tissu (11, 21).

4. Le pansement pour blessures selon les revendications 1 et 2, dans lequel ladite couche de tissu (11, 21) est composée d'un polyester filé-agglutiné, filé-entrelacé ou piqué-agglutiné.

5. Le pansement pour blessures selon les revendications 1 et 2, dans lequel les composants dudit pansement pour blessures ont tous une vitesse de transmission de vapeur humide lors de la mesure effectuée selon le procédé ASTM N° E96-80 d'au moins 200 grammes d'eau, de préférence, d'au moins 300 grammes d'eau par mètre carré en 24 heures, à 36°C, dans une humidité relative de 50%.

6. Le pansement pour blessures selon les revendications 1 et 2, dans lequel ledit pansement comprend une couche détachable (16, 25), collée de façon détachable sur la surface exposée de ladite couche adhésive (12, 22).

7. Le pansement pour blessures selon les revendications 1 et 2, dans lequel ledit pansement pour blessures comprend une couche de matériau cellulaire souple, à cellules ouvertes, disposée entre ladite couche de tissu (11, 21) et la couche de recouvrement (17, 27) et est collée à celle-ci par un adhésif.

8. Le procédé de fabrication d'un pansement pour blessures composé d'une couche de tissu, d'une couche d'adhésif sensible à la pression appliquée sur celle-ci et collée sur la face en largeur de ladite couche de tissu, une couche détachable disposée sur ladite couche adhésive et collée à celle-ci de façon détachable, une seconde couche adhésive disposée sur l'autre face en largeur de ladite couche de tissu et collée à celle-ci, une couche souple de matériau hydrophile disposée dans ladite couche de tissu, entre lesdites première et seconde couches de matériau adhésif et une couche de recouvrement disposée de manière à former la face distale dudit pansement pour plaies, ledit procédé comprenant :
a. la combinaison par collage d'une feuille dudit matériau de la couche de tissu et d'une feuille dudit matériau de la couche détachable avec une feuille intermédiaire de matériau de ladite première couche adhésive,
b. l'introduction d'une couche de matériau à structure hydrophile dans ladite couche de tissu et la transformation de ladite couche de matériau à structure hydrophile en une couche hydrophile disposée dans ladite couche de tissu,
c. l'application de ladite seconde couche de matériau adhésif, par collage, sur la face en largeur exposée de la couche de tissu, et
d. l'assemblage de ladite couche de recouvrement de manière à former le composant distal dudit pansement pour blessures.

9. Le procédé selon la revendication 8, dans lequel une couche de matériau cellulaire à cellules ouvertes est disposée par collage entre ladite couche de recouvrement et ladite seconde couche de matériau adhésif.

10. Le procédé selon la revendication 8, dans lequel on utilise l'énergie de rayonnement pour polymériser ledit matériau à structure hydrophile de manière à produire une couche de matériau hydrophile.

11. Le procédé selon la revendication 8, dans lequel ladite couche de recouvrement est appliquée par collage sur la seconde couche de matériau adhésif.

## Revendications (Revendications pour l'(les) Etat(s) contractant(s) suivant(s): ES)

1. Procédé de fabrication d'un pansement pour blessures pour le contrôle des fluides de la blessure par une absorption rapide des exsudats de la plaie et de manière à minimiser ainsi la macération de la peau, ledit pansement comprenant un stratifié contenant les souches suivantes :
(a) une couche adhésive (12, 22) susceptible de laisser passer le fluide de la blessure au travers, ladite couche adhésive, lors de son contact par pression sur la peau d'un patient, assurant une adhérence prolongée du pansement pour blessures sur ladite peau, sans adhérer à la plaie ;
(b) une couche de tissu (11, 21) collée sur ladite couche adhésive (12, 22), ladite couche de tissu conservant son intégrité structurelle lors de l'exposition dudit pansement pour blessures à l'exsudat de la plaie ; et
(c) une couche de recouvrement (17, 27) formant la surface distale dudit pansement pour blessures,
ledit pansement pour blessures étant caractérisé en ce qu'il comporte une couche de polymère absorbant hydrophile (15, 24) comprenant un matériau absorbant hydrophile déposé essentiellement en totalité dans ladite couche de tissu (11, 21), ladite couche de polymère absorbant hydrophile étant susceptible d'absorber le liquide attiré dans ledit pansement pour blessures, provenant dudit exsudat de la plaie, lorsque ledit pansement est appliqué sur la plaie d'un patient, ledit procédé comprenant :
a. la combinaison par collage d'une feuille dudit matériau de la couche de tissu et d'une feuille dudit matériau de la couche détachable avec une feuille intermédiaire de matériau de ladite première couche adhésive,
b. l'introduction d'une couche de matériau à structure hydrophile dans ladite couche de tissu et la transformation de ladite couche de matériau à structure hydrophile en une couche hydrophile disposée dans ladite couche de tissu,
c. l'application de ladite seconde couche de matériau adhésif, par collage, sur la face en largeur exposée de la couche de tissu, et
d. l'assemblage de ladite couche de recouvrement de manière à former l'élément distal dudit pansement pour plaies.

2. Le procédé selon la revendication 1, dans lequel ladite couche de tissu (11, 21) comporte des fibres en saillie noyées à l'intérieur et liées mécaniquement à ladite couche adhésive (12, 22).

3. Le procédé selon les revendications 1 et 2, dans lequel ledit matériau absorbant hydrophile est une résine acrylique réticulée et est durci par une solution de monomère disposée dans ladite couche de tissu (11, 21).

4. Le procédé selon les revendications 1 et 2, dans lequel ladite couche de tissu (11, 21) est composée d'un polyester filé-agglutiné, filé-entrelacé ou piqué-agglutiné.

5. Le procédé selon les revendications 1 et 2, dans lequel les composants dudit pansement pour blessures ont tous une vitesse de transmission de vapeur humide lors de la mesure effectuée selon le procédé ASTM N° E96-80 d'au moins 200 grammes d'eau, de préférence, d'au moins 300 grammes d'eau par mètre carré en 24 heures, à 36°C, dans une humidité relative de 50%.

6. Le procédé selon les revendications 1 et 2, dans lequel ledit pansement pour blessures comprend une couche release (16, 25), collée de façon amovible sur la surface exposée de ladite couche adhésive (12, 22).

7. Le procédé selon les revendications 1 et 2, dans lequel ledit pansement pour blessures comprend une couche de matériau cellulaire souple, à cellules ouvertes, disposée entre ladite couche de tissu (11, 21) et la couche de recouvrement (17, 27) et est collée à celle-ci par un adhésif.

8. Le procédé de fabrication d'un pansement pour blessures composé d'une couche de tissu, d'une couche d'adhésif sensible à la pression appliquée sur celle-ci et collée sur la face en largeur de ladite couche de tissu, une couche détachable disposé sur ladite couche adhésive et collée à celle-ci de façon détachable, une seconde couche adhésive disposée sur l'autre face en largeur de ladite couche de tissu et collée à celle-ci, une couche souple de matériau hydrophile disposée dans ladite couche de tissu, entre lesdites première et seconde couches de matériau adhésif et une couche de recouvrement disposée de manière à former la face distale dudit pansement pour blessures, ledit procédé comprenant :
a. la combinaison par collage d'une feuille dudit matériau de la couche de tissu et d'une feuille dudit matériau de la couche détachable avec une feuille intermédiaire de matériau de ladite première couche adhésive,
b. l'introduction d'une couche de matériau à structure hydrophile dans ladite couche de tissu et la transformation de ladite couche de matériau à structure hydrophile en une couche hydrophile disposée dans ladite couche de tissu,
c. l'application de ladite seconde couche de matériau adhésif, par collage, sur la face en largeur exposée de la couche de tissu, et
d. l'assemblage de ladite couche de recouvrement de manière à former le composant distal dudit pansement pour plaies.

9. Le procédé selon la revendication 8, dans lequel une couche de matériau cellulaire à cellules ouvertes est disposée par collage entre ladite couche de recouvrement et ladite seconde couche de matériau adhésif.

10. Le procédé selon la revendication 8, dans lequel on utilise l'énergie de rayonnement pour polymériser ledit matériau de structure hydrophile de manière à produire une couche de matériau hydrophile.

11. Le procédé selon la revendication 8 dans lequel ladite couche de recouvrement est appliquée par collage sur la seconde couche de matériau adhésif.
